(19) Europäisches Patentamt
European Patent Office
Office européen des brevets

(11) **EP 3 669 839 B1**

(12) **FASCICULE DE BREVET EUROPEEN**

(45) Date de publication et mention de la délivrance du brevet:
**08.09.2021 Bulletin 2021/36**

(51) Int Cl.:
*A61F 13/00* *(2006.01)*          *A41D 27/13* *(2006.01)*

(21) Numéro de dépôt: **19214204.0**

(22) Date de dépôt: **06.12.2019**

(54) **PIECE PROTECTRICE APTE A ABSORBER LA TRANSPIRATION, A REDUIRE LES ODEURS DE SUEUR ET A LIMITER L'APPARITION D'AUREOLES**

SCHUTZTEIL ZUR SCHWEISSABSORPTION, REDUZIERUNG DES SCHWEISSGERUCHS UND BESCHRÄNKUNG DES AUFTRETENS VON SCHWEISSFLECKEN

PROTECTIVE PART CAPABLE OF ABSORBING PERSPIRATION, REDUCING THE ODOUR OF SWEAT AND LIMITING THE APPEARANCE OF STAINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.12.2018 FR 1873448**

(43) Date de publication de la demande:
**24.06.2020 Bulletin 2020/26**

(73) Titulaire: **D.S.B.**
**59100 Roubaix (FR)**

(72) Inventeur: **DUCEPT, Sylvie**
**59166 Bousbecque (FR)**

(74) Mandataire: **Matkowska, Franck**
**Matkowska & Associés**
**9, rue Jacques Prévert**
**59650 Villeneuve d'Ascq (FR)**

(56) Documents cités:
WO-A1-2010/004175     WO-A2-2015/148758
FR-A1- 2 653 789     FR-A1- 3 026 923

## Description

### Domaine technique

La présente invention concerne une pièce protectrice qui est destinée à être en contact avec la peau en étant intégrée à un article d'habillement, et notamment à un sous-vêtement, et qui est apte à absorber la transpiration, à réduire les odeurs de sueur, et à limiter l'apparition d'auréoles. Cette pièce protectrice peut par exemple, et de manière non limitative, être une pièce axillaire.

### Art antérieur

Un problème récurrent dans les articles d'habillement, et notamment dans les articles d'habillement destinés à être portés en contact avec la peau, tels que des sous-vêtements, est lié à l'absorption par l'article d'habillement de la sueur produite par la peau du porteur de l'article d'habillement, en particulier dans toutes les zones du corps où cette production de sueur est la plus importante, tel que par exemple, mais pas exclusivement, les aisselles.

Cette absorption de la sueur par l'article d'habillement peut se traduire par l'émanation d'odeurs de sueur désagréables, par l'apparition préjudiciable d'auréoles de transpiration sur le côté extérieur (visible) de l'article d'habillement, et localement par une sensation d'humidité très désagréable pour le porteur de l'article d'habillement.

Pour réduire tout ou partie de ces phénomènes on a déjà proposé depuis de nombreuses années d'intégrer, de manière amovible ou non amovible, dans les articles d'habillement, notamment ceux destinés à être portés en contact avec la peau, tels que des sous-vêtements, des pièces protectrices dans les parties de l'article d'habillement correspondant aux zones du corps humain dans lesquelles la production de sueur est la plus importante, tel que par exemple les aisselles.

On a par exemple proposé dans la demande de brevet internationale WO2015/148758, une pièce protectrice absorbant la transpiration, et en particulier une pièce protectrice axillaire, qui est constituée d'une structure textile multicouche. Cette structure textile multicouche comporte au moins :

- une couche textile interne, par exemple un tricot, un tissu ou un non-tissé, qui est destinée à être au contact de la peau, et qui est constituée de fibres hydrophobes, ayant plus particulièrement un taux de reprise inférieur à 2% telles que par exemple des fibres en polyester ;
- une couche textile externe, par exemple un tricot, un tissu ou un non-tissé, qui présente des propriétés d'absorption supérieure à la couche textile interne de manière à absorber la transpiration et qui peut sécher rapidement afin d'éviter une saturation de cette couche par la transpiration ; cette couche textile externe est constituée de fibres hydrophiles, et par exemple des fibres de coton, ou un mélange de fibres de coton et de polyester.

L'utilisation de fibres hydrophobes, telles que des fibres en polyester, dans la couche textile interne destinée à être en contact avec la peau, se traduit en pratique par une très faible absorption de la sueur par la pièce de protection et par une macération, entre la peau et cette couche textile interne, d'une partie de la sueur produite, aboutissant dans le temps à la production d'odeurs de sueur particulièrement désagréables. En effet, les fibres étant hydrophobes, elles n'absorbent pas la sueur, ni les molécules odorantes, qui restent donc en surface du textile. Ces molécules odorantes sont donc très peu neutralisées, d'où le développement d'odeurs sur la face du textile en contact peau. En outre, les fibres dans la face du textile en contact avec la peau étant hydrophobes, il subsiste à l'usage une sensation d'humidité très désagréable en cas de sudation.

On a également déjà proposé dans la demande de brevet internationale WO 2010/004175, une pièce protectrice absorbant la transpiration, et en particulier une pièce protectrice axillaire, comportant un tricot 3D, dans lequel :

- la face interne du tricot 3D, pouvant être en contact avec la peau, est par exemple constituée de fibres ou filaments aptes à assurer un transfert humidité par capillarité, et sont par exemple des fibres polyester « COOLMAX® » ;
- la face externe du tricot 3D est par exemple à base de fils polyester ;
- les fils reliant les deux faces interne et externe du tricot 3D sont dans un matériau absorbant, et sont par exemple des fils à base de viscose, de préférence ayant des propriétés bactériostatiques (viscose traitée bactériostatique ou associée à un fil d'argent).

Comparativement aux fibres polyester hydrophobes de la couche textile interne de la demande de brevet internationale WO2015/148758 susvisée, l'utilisation de fibres polyester « COOLMAX® » permet certes d'améliorer le transfert d'humidité, mais en pratique en cas de sudation une sensation d'humidité désagréable subsiste néanmoins. En outre l'utilisation de fils à base de viscose, traités pour avoir des propriétés bactériostatiques, permet de réduire la prolifération bactériologique à l'origine des odeurs de sueur à l'intérieur du tricot 3D, mais ne permet pas de réduire l'émanation d'odeur entre la peau et la face interne du tricot 3D.

Surtout, les couches de ce tricot 3D, et en particulier la face interne de ce tricot 3D à base de fils polyester, ne permet pas de réduire efficacement les odeurs de sueur produites.

Au surplus, dans cette demande de brevet internationale WO2015/148758, il est préconisé de mettre en oeuvre une couche externe supplémentaire imperméable, qui est par exemple constituée par une enduction adhésive ou qui est par exemple constituée d'une membrane (ou film) imperméable et de préférence imper-respirante.

La mise en œuvre de cette couche externe supplémentaire imperméable, constituée par exemple d'une membrane (ou film) imperméable et imper-respirante, permet certes de réduire l'apparition d'auréoles sur la face externe de la pièce de protection, mais conduit à bloquer la sueur produite par la peau, de manière beaucoup trop importante dans le tricot 3D et entre ce tricot 3D et la peau, ce qui contribue en définitive à augmenter de manière préjudiciable la macération de cette sueur, et aboutit dans le temps au développement d'odeurs de sueur désagréables sur la face interne, malgré la mise en œuvre de fils à base de viscose, traités pour avoir des propriétés bactériostatiques.

On a également déjà proposé dans la demande de brevet français FR3026923 un vêtement comportant une ou plusieurs pièces de protection axillaires amovibles (« patchs »). Chaque pièce de protection est constituée de trois couches :

- une première couche qui est destinée à être en contact avec la peau mais n'est pas absorbante et est constituée par un tissu antibactérien, plus particulièrement un « tissu intégrant une fibre X-Static® en fibres d'argent » ;
- une deuxième couche qui est un tissu absorbant ;
- une troisième couche décrite comme une couche imperméable, *« qui empêche totalement la sueur absorbée par les couches internes de mouiller les couches externes apparentes ».*

**Objectif de l'invention**

Un objectif de l'invention est de proposer une nouvelle pièce protectrice, qui est destinée à être en contact avec la peau en étant intégrée à un article d'habillement, et notamment à un sous-vêtement, et qui permet d'absorber efficacement la transpiration tout en réduisant les odeurs de sueur, et de limiter l'apparition d'auréoles sur sa face externe orientée vers l'article d'habillement.

**Résumé de l'invention**

L'invention a ainsi pour premier objet une pièce protectrice destinée à être intégrée à un article d'habillement, et notamment à un sous-vêtement, ladite pièce protectrice étant apte à absorber la transpiration, à réduire les odeurs de sueur, et à limiter l'apparition d'auréoles ; ladite pièce protectrice est perméable à l'air et au moins à la vapeur d'eau et comporte deux structures textiles superposées:

- une première structure textile absorbante comportant au moins un premier tricot ou tissu absorbant, dont la face interne constitue la face interne de la pièce protectrice destinée à être en contact avec la peau, et qui comporte sur sa face interne majoritairement des fils hydrophiles aptes à absorber l'humidité et à réduire les odeurs de sueur, ladite première structure textile absorbante présentant un taux de réduction d'odeur, mesuré par la méthode ISO 17299-3 de dosage de l'acide acétique par chromatographie en phase gazeuse (acide acétique déposée sur ladite face interne), qui est supérieur à 50%, et
- une deuxième structure textile formant une barrière à l'humidité et comportant au moins un deuxième tricot ou tissu hydrophobe en contact avec ladite première structure textile absorbante, ladite deuxième structure textile étant perméable à l'air et respirante avec une résistance à la vapeur d'eau ($R_{et}$) en régime stationnaire, mesurée selon la norme NF EN ISO 11092:2014, inférieure à 6 $m^2$.Pa/W,

ladite pièce protectrice étant dépourvue de couche supplémentaire présentant une résistance à la vapeur d'eau ($R_{et}$) en régime stationnaire, mesurée selon la norme NF EN ISO 11092:2014, supérieure à 6 $m^2$.Pa/W.

Par le terme « majoritairement », on signifie que lesdits fils hydrophiles constituent au moins 50% des fils présents sur la face interne du premier tricot ou tissu absorbant.

Un taux de réduction d'odeur faible signifie que la structure textile ne retient pas ou peu l'acide acétique, cette substance odorante étant facilement libérée dans l'air. A contrario, un taux de réduction d'odeur élevé signifie que la structure textile retient fortement l'acide acétique et que celui-ci est peu libéré dans l'air, d'où une émanation d'odeur faible, car le composé odorant reste piégé (imprégné) dans la structure textile.

Dans le présent texte, par les termes « *réduire les odeurs de sueur* », on signifie ainsi que les fils hydrophiles ou la structure et textile sont aptes à piéger au moins en partie l'acide acétique (substance odorante), et permettent ainsi de réduire l'émanation d'odeur de sueur. On considère également dans le présent texte que les termes « *neutraliser les odeurs de sueur»* ou « *neutralisation des odeurs de sueur »* ont la même signification respectivement que« *réduire les odeurs de sueur »* et *«réduction des odeurs de sueur ».*

La mesure de la résistance à la vapeur d'eau ($R_{et}$) en régime stationnaire d'une structure textile ou couche textile, et en particulier de la deuxième structure textile de la pièce protectrice susvisée, est réalisée de manière usuelle en mettant en œuvre la méthode de mesure de la norme NF EN ISO 11092 : 2014.

Plus particulièrement, la pièce protectrice peut comporter les caractéristiques additionnelles et facultatives ci-après, prises isolément ou en combinaison les unes avec les autres :

- lesdits fils hydrophiles aptes à absorber l'humidité et à réduire les odeurs de sueur comportent des fils comportant une ou plusieurs matières naturelles hydrophiles, et de préférence au moins 50% en poids de une ou plusieurs matières naturelles hydrophiles, et plus préférentiellement encore 100% en poids de matières naturelles hydrophiles.
- la ou les matières naturelles hydrophiles sont choisies parmi les matières de la liste suivante : coton, lin, soie, laine, ou un mélange d'au moins deux de ces matières.
- lesdits fils hydrophiles aptes à absorber l'humidité et à réduire les odeurs de sueur comportent des fils synthétiques traités hydrophiles.
- lesdits fils hydrophiles ou le premier tricot ou tissu ont été traités avec au moins un agent bactériostatique.
- lesdits fils hydrophiles aptes à absorber l'humidité et à réduire les odeurs de sueur comportent des fils comportant du polyester chargé en carbone, et de préférence chargé en charbon de bambou, de préférence des fils comportant au moins 50% en poids de polyester chargé en carbone, et plus préférentiellement des fils comportant 100% en poids de polyester chargé en carbone.
- ledit premier tricot ou tissu absorbant comporte majoritairement sur sa face interne lesdits fils hydrophiles aptes à absorber l'humidité et à réduire les odeurs de sueur, et comporte majoritairement sur son autre face externe des fils hydrophobes, de manière à former une première barrière à l'humidité en complément de la deuxième structure textile.
- lesdits fils hydrophobes présents majoritairement sur la face externe du premier tricot ou tissu absorbant comportent des fils hydrophobes à base de polyester, de préférence des fils 100% polyester.

L'invention a également pour deuxième objet une pièce protectrice destinée à être intégrée à un article d'habillement, et notamment à un sous-vêtement, ladite pièce protectrice étant apte à absorber la transpiration, à réduire les odeurs de sueur, et à limiter l'apparition d'auréoles, ladite pièce protectrice étant perméable à l'air et au moins à la vapeur d'eau et comportant deux structures textiles superposées:

- une première structure textile absorbante comportant au moins un premier tricot ou tissu absorbant, dont la face interne constitue la face interne de la pièce protectrice destinée à être en contact avec la peau, qui comporte sur sa face interne majoritairement des fils hydrophiles aptes à absorber l'humidité et à réduire les odeurs de sueur, et qui comporte majoritairement sur son autre face externe des fils hydrophobes, de manière à former une première barrière à l'humidité en complément de la deuxième structure textile, et
- une deuxième structure textile formant une barrière à l'humidité et comportant au moins un deuxième tricot ou tissu hydrophobe en contact avec ladite première structure textile absorbante.

Plus particulièrement, cette pièce protectrice peut comporter les caractéristiques additionnelles et facultatives ci-après, prises isolément ou en combinaison les unes avec les autres :

- ladite première structure textile absorbante présente un taux de réduction d'odeur, mesuré par la méthode ISO 17299-3 de dosage de l'acide acétique par chromatographie en phase gazeuse, qui est supérieur à 50%.
- ladite deuxième structure textile présente une résistance à la vapeur d'eau (Ret) en régime stationnaire, mesurée selon la norme NF EN ISO 11092 :2014, inférieure à 6 $m^2$.Pa/W.
- la pièce protectrice est dépourvue de couche supplémentaire présentant une résistance à la vapeur d'eau ($R_{et}$) en régime stationnaire, mesurée selon la norme NF EN ISO 11092:2014, supérieure à 6 $m^2$.Pa/W.
- lesdits fils hydrophiles aptes à absorber l'humidité et à réduire les odeurs de sueur comportent des fils comportant une ou plusieurs matières naturelles hydrophiles, et de préférence au moins 50% en poids de une ou plusieurs matières naturelles hydrophiles, et plus préférentiellement encore 100% en poids de matières naturelles hydrophiles.
- la ou les matières naturelles hydrophiles sont choisies parmi les matières de la liste suivante : coton, lin, soie, laine, ou un mélange d'au moins deux de ces matières.
- lesdits fils hydrophiles aptes à absorber l'humidité et à réduire les odeurs de sueur comportent des fils synthétiques traités hydrophiles.

- lesdits fils hydrophiles ou le premier tricot ou tissu ont été traités avec au moins un agent bactériostatique.
- lesdits fils hydrophiles aptes à absorber l'humidité et à réduire les odeurs de sueur comportent des fils comportant du polyester chargé en carbone, et de préférence chargé en charbon de bambou, de préférence des fils comportant au moins 50% en poids de polyester chargé en carbone, et plus préférentiellement des fils comportant 100% en poids de polyester chargé en carbone.
- lesdits fils hydrophobes présents majoritairement sur la face externe du premier tricot ou tissu absorbant comportent des fils à base de polyester, de préférence des fils 100% polyester.

L'invention a ainsi pour troisième objet une pièce protectrice destinée à être intégrée à un article d'habillement, et notamment à un sous-vêtement, ladite pièce protectrice étant apte à absorber la transpiration, à réduire les odeurs de sueur, et à limiter l'apparition d'auréoles, ladite pièce protectrice étant perméable à l'air et au moins à la vapeur d'eau et comportant deux structures textiles superposées :

- une première structure textile absorbante comportant au moins un premier tricot ou tissu absorbant, dont la face interne constitue la face interne de la pièce protectrice destinée à être en contact avec la peau, et qui comporte sur sa face interne majoritairement des fils hydrophiles qui sont aptes à absorber l'humidité et à réduire les odeurs de sueur, lesdits fils hydrophiles comportant une ou plusieurs matières naturelles hydrophiles, et de préférence au moins 50% en poids de une ou plusieurs matières naturelles hydrophiles, et plus préférentiellement encore 100% en poids de matières naturelles hydrophiles, la ou les matières naturelles hydrophiles étant choisies parmi les matières de la liste suivante: coton, lin, soie, laine, ou un mélange d'au moins deux de ces matières et/ou lesdits fils hydrophiles comportant des fils synthétiques traités hydrophiles et comportant du polyester chargé en carbone, et de préférence chargé en charbon de bambou, de préférence des fils comportant au moins 50% en poids de polyester chargé en carbone, et plus préférentiellement des fils comportant 100% en poids de polyester chargé en carbone,
- et
- une deuxième structure textile formant une barrière à l'humidité et comportant au moins un deuxième tricot ou tissu (210) hydrophobe en contact avec ladite première structure textile absorbante (21), ladite deuxième structure textile étant perméable à l'air et respirante avec une résistance à la vapeur d'eau (Ret) en régime stationnaire, mesurée selon la norme NF EN ISO 11092:2014, inférieure à 6 $m^2$.Pa/W,

ladite pièce protectrice étant dépourvue de couche supplémentaire présentant une résistance à la vapeur d'eau ($R_{et}$) en régime stationnaire, mesurée selon la norme NF EN ISO 11092:2014, supérieure à 6 $m^2$.Pa/W.
Plus particulièrement, cette pièce protectrice peut comporter les caractéristiques additionnelles et facultatives ci-après, prises isolément ou en combinaison les unes avec les autres :

- ladite première structure textile absorbante présente un taux de réduction d'odeur, mesuré par la méthode ISO 17299-3 de dosage de l'acide acétique par chromatographie en phase gazeuse, qui est supérieur à 50%.
- ledit premier tricot ou tissu absorbant comporte majoritairement sur sa face interne lesdits fils hydrophiles aptes à absorber l'humidité et à réduire les odeurs de sueur, et comporte majoritairement sur son autre face externe des fils hydrophobes, de manière à former une première barrière à l'humidité en complément de la deuxième structure textile.
- lesdits fils hydrophobes présents majoritairement sur la face externe du premier tricot ou tissu absorbant comportent des fils à base de polyester, de préférence des fils 100% polyester.

Plus particulièrement, chacune des pièces protectrice susvisées peut comporter les caractéristiques additionnelles et facultatives ci-après, prises isolément ou en combinaison les unes avec les autres :

- ladite première structure textile absorbante présente un taux de réduction d'odeur supérieur à 60%, de préférence supérieur à 70%.
- ladite première structure textile absorbante est constituée uniquement dudit premier tricot ou tissu absorbant.
- ladite deuxième structure textile formant une barrière à l'humidité est constituée uniquement dudit deuxième tricot ou tissu hydrophobe.
- la pièce protectrice est constituée uniquement de ladite première structure textile absorbante et de ladite deuxième structure textile formant une barrière à l'humidité.
- La pièce protectrice est constituée uniquement dudit premier tricot ou tissu absorbant et dudit deuxième tricot ou tissu hydrophobe.
- le premier tricot ou tissu absorbant comporte au moins un fil élastique, et plus particulièrement un fil en élasthanne.
- la résistance à la vapeur d'eau (Ret) en régime stationnaire, mesurée selon la norme NF EN ISO 11092:2014, de ladite deuxième structure textile est inférieure à 4,5 m$^2$.Pa/W, de préférence inférieure à 3,5 m$^2$.Pa/W, et plus préférentiellement inférieure à 2 m$^2$.Pa/W.

- la première structure textile absorbante présente une résistance à la vapeur d'eau (Ret) en régime stationnaire, mesurée selon la norme NF EN ISO 11092:2014, inférieure à 6 m$^2$.Pa/W, de préférence inférieure à 4,5 m$^2$.Pa/W, et plus préférentiellement inférieure à 3 m$^2$.Pa/W.
- la première structure textile absorbante présente un taux de reprise supérieur à 4%, de préférence supérieur à 5%, et plus préférentiellement encore supérieur à 6%.
- la deuxième structure textile présente un taux de reprise mesuré inférieur à 3%.
- la première structure textile absorbante a une perméabilité à l'air supérieure à 100 l/m$^2$/s, et de préférence supérieure à 200 l/m$^2$/s.
- la deuxième structure textile a une perméabilité à l'air supérieure à 300/m$^2$/s, de préférence supérieure à 600 l/m$^2$/s, et plus préférentiellement encore supérieure à 800 l/m$^2$/s.
- la pièce protectrice a une perméabilité à l'air supérieure à 100 l/m$^2$/s, et de préférence supérieure à 200 l/m$^2$/s.
- le deuxième tricot ou tissu hydrophobe comporte des fils hydrophobes synthétiques, notamment des fils à base polypropylène, de préférence des fils 100% polypropylène et/ou des fils à base de polyester, de préférence des fils 100% polyester.

L'invention a également pour objet un article d'habillement, et notamment sous-vêtement, dans lequel est intégrée, de manière amovible ou non amovible, au moins une pièce protectrice susvisée, de telle sorte que la face interne du premier tricot ou tissu absorbant peut être en contact avec la peau.

L'invention a également pour objet un article d'habillement pour partie haute du corps, et notamment sous-vêtement, dans lequel sont intégrées, de manière amovible ou non amovible, au niveau respectivement des deux aisselles, deux pièces axillaires constituées de pièces protectrices susvisées, de telle sorte que la face interne du premier tricot ou tissu absorbant de chaque pièce protectrice axillaire peut être en contact avec la peau.

## Brève description des dessins

Les caractéristiques et avantages de l'invention apparaîtront plus clairement à la lecture de la description détaillée ci-après de plusieurs variantes particulières de réalisation de l'invention, lesquels variantes particulières de réalisation sont décrites à titre d'exemples non limitatifs et non exhaustifs de l'invention et en référence aux dessins annexés sur lesquels :

- la figure 1 représente de manière schématique un sous-vêtement de type tee-shirt, pourvu de deux pièces axillaires protectrices conformes à l'invention,
- la figure 2 est un exemple préféré de structure pour le tricot absorbant d'une pièce protectrice de l'invention.

## Description détaillée

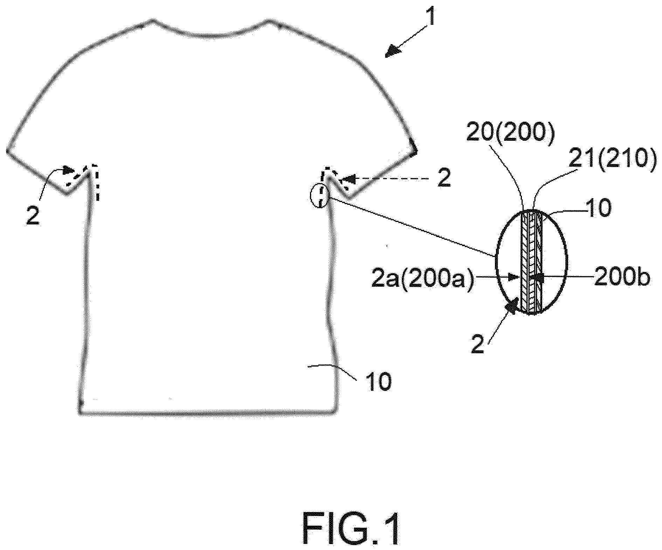

On a représenté sur la figure 1, un exemple d'article d'habillement 1, en l'espèce un sous-vêtement, de type tee-shirt, qui est destiné à être porté en contact avec la peau, et à l'intérieur duquel sont fixées deux pièces protectrices 2, qui sont conformes à l'invention, et qui permettent d'absorber la transpiration, de réduire les odeurs de sueur, et de limiter l'apparition d'auréoles dans l'article d'habillement 1.

Le matériau 10 constitutif de l'article d'habillement 1 est sans importance pour l'invention et dépend du type de l'article d'habillement.

Dans cet exemple particulier, mais de manière non limitative de l'invention, chaque pièce protectrice 2 est une pièce axillaire fixée dans la région d'une aisselle du sous-vêtement 1, étant précisé que dans le cadre de l'invention, une pièce protectrice peut être utilisée avec tout type d'article d'habillement, en étant positionnée dans toute partie d'un article d'habillement 1, dans laquelle l'on souhaite absorber la sueur produite localement par la peau du porteur de l'article d'habillement 1.

Dans le cadre de l'invention, la pièce protectrice 2 peut être une pièce rapportée, qui est fixée de manière définitive et non amovible à l'article d'habillement 1, par exemple en étant cousue et/ou collée sur sa périphérie à l'intérieur de l'article d'habillement 1. La pièce protectrice 2 peut être une pièce rapportée, qui est solidarisée de manière amovible à l'article d'habillement 1, par exemple au moyen d'un système de fixation à boucles et crochets type VELCRO®. La pièce protectrice 2 peut également être une pièce faisant partie intégrante de l'article d'habillement et par exemple une pièce constituant la partie axillaire ou autre de l'article d'habillement.

La géométrie de la pièce protectrice 2 est sans importance pour l'invention, la pièce de protection pouvant par exemple, et de manière non exhaustive, avoir une forme de disque, une forme polygonale, une forme bilobée,... Quelle que soit sa géométrie, la surface de la pièce protectrice 2 doit être suffisante pour couvrir la zone de sudation de la peau que l'on cherche à protéger (par exemple les aisselles).

En référence à la figure 1, la pièce protectrice 2 est perméable à l'air et au moins à la vapeur d'eau et est constituée dans cet exemple particulier de deux structures textiles 20, 21 superposées en contact l'une de l'autre, à savoir:

- une première structure textile 20 absorbante et apte à réduire les odeurs de sueur,
- une deuxième structure textile 21 en contact avec ladite première structure textile absorbante 20 et formant une barrière à l'humidité.

**Première structure textile 20 absorbante et apte à capturer les odeurs de sueur**

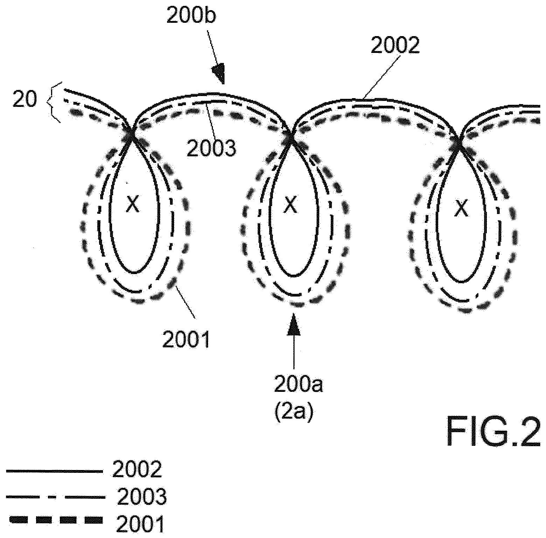

La première structure textile absorbante 20 comporte au moins un premier tricot ou tissu absorbant 200 (cf. par exemple figure 2), dont la face interne 200a constitue la face interne 2a de la pièce protectrice 2 et est destinée à être en contact avec la peau.

Dans l'exemple de la figure 1, la face interne 200a du premier tricot ou tissu 200 absorbant de chaque pièce protectrice axillaire 2 est donc orientée vers l'intérieur de l'article d'habillement 1.

Ce premier tricot ou tissu absorbant 200 comporte sur sa face interne 200a majoritairement des fils hydrophiles aptes à absorber l'humidité et à capturer les odeurs de sueur.

Ladite première structure textile absorbante 20 présente par ailleurs un taux de réduction d'odeur, mesuré par la méthode ISO 17299-3 de dosage de l'acide acétique par chromatographie en phase gazeuse, qui est supérieur à 50%, de préférence un taux de réduction d'odeur supérieur à 60%, et plus préférentiellement un taux de réduction d'odeur supérieur à 70%.

Mesure du taux de réduction d'odeur

Pour mesurer le taux de réduction d'odeur de la première structure textile absorbante 20 de la pièce protectrice 2 de l'invention, on met en œuvre la méthode ISO 17299-3 de dosage de l'acide acétique par chromatographie en phase gazeuse.

À cet effet, on utilise une solution test qui contient 9g/l de NaCl (Chlorure de Sodium) et 1 g/l d'acide acétique.

On prépare des échantillons de 5cm x 5cm de la première structure textile absorbante 20 à tester.

On dépose 850 µl de cette solution sur chaque échantillon à tester, sur la face interne 200a destinée à être en contact avec la peau.

Les échantillons sont placés dans des flacons (« vials) fermés hermétiquement. Trois réplicas sont préparés pour chaque échantillon.

Un flacon étalon (solution test sans tricot ou tissu) est intercalé de manière régulière entre chaque série d'échantillons testés.

Les flacons sont chauffés à 80°C pendant 30 minutes à l'aide d'un « headspace » et 1ml de la phase gazeuse ainsi générée est injecté dans le chromatographe en phase gazeuse couplé à un détecteur FID. Le débit dans la colonne du chromatographe en phase gazeuse est de 1,03421 bar (15 psi). Le débit du flacon (« vial ») est de 0,344738 bar (5psi).

L'aire du pic chromatographique d'acide acétique détecté par FID est proportionnelle à la quantité d'acide acétique présente dans la phase gazeuse du flacon (« vial ») (c'est-à-dire le gaz d'essai selon la terminologie de la norme)

Le taux de réduction d'odeur p est donné par la formule suivante :

$$\rho = \frac{(A0 - A1)\ \text{x}\ 100}{A0}$$

dans laquelle A0 est l'aire de pic moyen de gaz d'essai sans tissu et A1 est l'aire de pic moyen de gaz d'essai avec tissu.

La capacité d'absorption de l'humidité des fils hydrophiles du premier tricot ou tissu 200 permet de drainer localement la sueur produite par le porteur de l'article d'habillement 1 et ainsi de conférer à ce porteur une sensation de « sec » sur sa peau. La capacité des fils hydrophiles du premier tricot ou tissu 200 à capturer les odeurs de sueur permet de piéger, dans ce premier tricot ou tissu, les molécules odorantes de la sueur, qui a été absorbée par ces fils hydrophiles, et ainsi de réduire les émanations de mauvaises odeurs par la pièce protectrice.

Dans le cadre de l'invention, ladite première structure textile 20 absorbant humidité et réduisant les odeurs de sueur peut comporter plusieurs couches textiles superposées, et notamment plusieurs tricots et/ou tissus superposés, dont au moins ledit premier tricot ou tissu 200 absorbant.

Néanmoins, dans une variante préférée de réalisation, ladite première structure textile absorbante 20 est constituée uniquement dudit premier tricot ou tissu absorbant 200. Dans ce cas ledit premier tricot ou tissu absorbant 200 présente un taux de réduction d'odeur, mesuré par la méthode ISO 17299-3 de dosage de l'acide acétique par chromatographie en phase gazeuse, qui est supérieur à 50%, de préférence un taux de réduction d'odeur supérieur à 60%, et plus préférentiellement un taux de réduction d'odeur supérieur à 70%.

*Caractère hydrophile des fils du premier tricot ou tissu 200*

Le caractère hydrophile des fils du premier tricot ou tissu 200 qui absorbent l'humidité et réduisent les odeurs de sueur peut découler intrinsèquement de la matière constitutive du fil. Il peut s'agir par exemple de fils naturels intrinsèquement hydrophiles et présentant naturellement des propriétés de neutralisation des odeurs de sueur.

Différents types de fils naturels intrinsèquement hydrophiles et aptes à neutraliser (ou réduire) les odeurs de sueur peuvent être utilisés pour réaliser le premier tricot ou tissu 200.

Par exemple, et de manière non limitative de l'invention, les fils hydrophiles du premier tricot ou tissu 20 peuvent comporter des fils naturels à base de coton ou de lin, et ou des fils à base de laine ou à base de soie.

De préférence, ces fils naturels hydrophiles à base de coton ou de lin, et ou ces fils naturels hydrophiles à base de laine ou à base de soie comportent au moins 50% en poids de ladite matière naturelle hydrophile (coton ou lin, ou soie ou laine).

Plus préférentiellement encore, ces fils naturels à base de coton ou de lin, et ou ces fils naturels hydrophiles à base de laine ou à base de soie comportent 100% en poids de ladite matière naturelle hydrophile (coton ou lin, ou soie ou laine).

Ces fils naturels hydrophiles peuvent également comporter un mélange d'au moins deux matières naturelles hydrophiles différentes choisies parmi lesdites matières naturelles hydrophiles susvisées.

Les fils hydrophiles du premier tricot ou tissu 200 peuvent également comporter des fibres naturelles hydrophiles (de préférence au moins 50% en poids), et en particulier des fibres de coton ou de lin, ou des fibres de laine ou des fibres de soie, mélangées à d'autres types de fibres ou microfibres tels que par exemple des fibres ou microfibres synthétiques, et notamment des fibres ou microfibres polyester pour une meilleure stabilité dimensionnelle du tricot ou tissu.

Les fils hydrophiles du premier tricot ou tissu 200 absorbant qui absorbent l'humidité et réduisent les odeurs de sueur peuvent également comporter des fils synthétiques, qui ont été traités au moyen d'agent(s) hydrophile(s) connus, de manière à être rendus hydrophiles, et qui ont des propriétés de neutralisation (ou réduction) des odeurs de sueur.

Ces fils en matière synthétique peuvent être avoir été rendus hydrophiles par ajouts d'agent(s) hydrophile(s) dans la composition du polymère constitutif des fils synthétiques ou par une enduction de surface des fils au moyen d'agent(s) hydrophile(s), cette enduction de surface pouvant entre réalisée au cours de l'extrusion à chaud de ces fils.

Les fils hydrophiles en matière synthétique du premier tricot ou tissu 200 peuventégalement avoir été rendus hydrophiles postérieurement à leur fabrication, par enduction ou imprégnation du premier tricot ou tissu 200 avec un ou plusieurs agents hydrophiles connus, notamment par foulardage du premier tricot ou tissu 200 à l'entrée d'une rame, après teinture et séchage.

Plus particulièrement les fils synthétiques hydrophiles du premier tricot ou tissu 200 peuvent être des fils traités hydrophiles et comportant du polyester chargé en carbone, et notamment en charbon (ou carbone) de bambou (« bamboo charcoal ») et plus particulièrement des fils traités hydrophiles à base de polyester chargé en carbone, et notamment en charbon de bambou. Par *« fils à base de polyester chargé en carbone »*, on désigne des fils comportant au moins 50% en poids de polyester chargé en carbone.

Typiquement, le polyester chargé en carbone, et notamment chargé en charbon de bambou comporte dans ce cas de préférence de 2 à 8% en poids de carbone, et notamment de charbon de bambou.

A la différence des fibres 100% polyester qui n'ont pas de propriété leur permettant de neutraliser les odeurs de sueur, les fils synthétiques traités hydrophile et à base de polyester chargé en carbone, et notamment à base de polyester chargé en charbon de bambou, permettent une réduction efficace des odeurs de sueur.

Préférentiellement, on utilisera des fils 100% polyester chargé en carbone, et notamment chargé en charbon de bambou, et traités hydrophiles.

Lorsque on utilise des fils synthétiques traités hydrophiles, tels que notamment des fils traités hydrophiles et comportant du polyester chargé en carbone, et notamment en charbon de bambou, il est préférable de traiter ces fils synthétiques et/ou ledit premier tricot ou tissu 200 avec un ou plusieurs agents bactériostatiques connus (par exemple ammonium quaternaire, zinc pyrithione,...), qui ont pour fonction de limiter le développement des bactéries à l'origine des odeurs de sueur. On améliore ainsi les propriétés de neutralisation des odeurs de ces fils synthétiques ayant subi un traitement hydrophile.

Les fils hydrophiles du premier tricot ou tissu 200 peuvent également comporter des fils en filé de fibres comportant des fibres naturelles hydrophiles (de préférence au moins 50% en poids), et en particulier des fibres de coton ou de lin, ou des fibres de laine ou des fibres de soie, mélangées à des fibres ou microfibres de polyester chargé en carbone, et de préférence de polyester chargé charbon de bambou. De préférence, mais non nécessairement, dans ce cas le poids des fibres naturelles peut être d'au moins 50% du poids total du fil et les fibres ou microfibres de polyester chargé en carbone, et notamment en charbon de bambou, ont subi un traitement hydrophile.

Le premier tricot ou tissu 200 peut également comporter plusieurs types différents de fils hydrophiles aptes à absorber l'humidité et à neutraliser les odeurs de sueur.

On notera par exemple qu'un tricot ou tissu constitué de 100% de fils polyester ne convient pas comme premier tricot ou tissu 200 car les fils polyester (sans traitement hydrophile) sont intrinsèquement hydrophobes et présentent un taux de reprise théorique inférieur à 2%. Surtout, à la différence des fils à base de polyester chargé en carbone ou des fils à base de de coton, les fils polyester n'ont pas de propriété leur permettant de neutraliser les odeurs de sueur. De même un tricot ou tissu constitué de 100% de fils en viscose ne convient pas comme premier tricot ou tissu 200 car les fils de viscose n'ont pas de propriété leur permettant de réduire suffisamment les odeurs de sueur, comparativement par exemple au coton.

De préférence, les fils hydrophiles du premier tricot ou tissu 200 sont choisis de telle sorte que le premier tricot ou tissu 200 présente un taux de reprise, caractérisant sa capacité d'absorption de l'humidité, qui est supérieur à 4%, de préférence supérieur à 5%, et plus préférentiellement encore supérieur à 6%.

Il est possible également dans le cadre de l'invention d'utiliser d'autres types de fils en combinaison avec les fils hydrophiles susvisés aptes à absorber l'humidité et à réduire les odeurs de sueur.

Par exemple, lorsque le premier tricot ou tissu 200 comporte des fils à base de coton, il peut être avantageux d'utiliser également des fils dans le tricot ou tissu qui ont pour fonction de stabiliser dimensionnellement le tricot ou tissu, par exemple des fils à base de polyester, et plus particulièrement des fils 100% polyester, afin notamment de permettre un lavage de la pièce protectrice 2 à des températures de lavage supérieures ou égales à 30°C, avec une rétraction du premier tricot ou tissu 200 inférieure à 5%-6%.

Le premier tricot ou tissu 200 peut également comporter des fils élastiques additionnels, de type fils en élasthanne ou fils synthétiques élastiques, de manière notamment à améliorer l'élasticité et la stabilité dimensionnelle du premier tricot ou tissu.

Dans le cadre de l'invention, le premier tricot ou tissu 200 peut être fabriqué en mettant en oeuvre tout type connu de structure de liage pour tricot ou tissu.

Néanmoins, lorsque le premier tricot ou tissu 200 comporte d'autres types de fils, tels que par exemple des fils à base de polyester, en combinaison avec les fils hydrophiles susvisés aptes à absorber l'humidité et à réduire les odeurs de sueur, il est préférable de mettre en œuvre une structure de tricotage ou tissage, qui permet de manière connue en soi d'obtenir un premier tricot ou tissu 200 dont la face interne 200a destinée à être en contact avec la peau, comporte majoritairement les fils hydrophiles susvisés aptes à absorber l'humidité et à réduire les odeurs de sueur, par exemple des fils à base de coton, et dont l'autre face externe 200b comporte majoritairement les autres types de fils, et par exemple des fils à base de polyester.

Dans ce cas, dans une variante préférée de réalisation, les fils présents majoritairement sur la face externe 200b du tricot ou tissu 200 sont de préférence des fils hydrophobes, de manière à former une première barrière à l'humidité en complément de l'action de barrière à l'humidité de la deuxième structure textile 21.

Le caractère hydrophobe de ces fils, positionnés majoritairement sur la face externe 200b du premier tricot ou tissu 200 peut découler intrinsèquement de la matière constitutive du fil. Il peut s'agir par exemple de fils synthétiques hydrophobes, tels que par exemple, mais de manière non limitative, des fils à base de polyester et notamment des fils 100% polyester.

Le caractère hydrophobe de ces fils peut également être obtenu ou renforcé au moyen d'agent(s) hydrophobe(s) connus ; par exemple des fils utilisables comme fils hydrophobes pour le premier tricot ou tissu 200, peuvent être avoir été rendus hydrophobes ou plus hydrophobes au moyen d'un traitement hydrophobe.

On a représenté sur la figure 2, un exemple préféré de réalisation d'un premier tricot 200, de type jersey vanisé, fabriqué à partir de trois types différents de fils à savoir :

- un fil hydrophile 2001 précédemment décrit, et par exemple un fil 100%coton,
- un fil 2002 hydrophobe, par exemple un fil 100% polyester,
- un fil élastique, de type fil élasthanne 2003, permettant de conférer de l'élasticité au tricot.

Grâce à cette structure de tricotage particulière, la face interne 200a du tricot 200 destinée à être en contact avec la peau, comprend majoritairement les fils hydrophiles 2001 et l'autre face externe 200b du tricot 200 comprend majoritairement les fils hydrophobes 2002.

## Deuxième structure textile 21 formant une barrière à l'humidité

La deuxième structure textile 21 comporte au moins un deuxième tricot ou tissu 210 en contact avec la première structure textile absorbante 20 susvisée, ledit deuxième tricot ou tissu 210 étant hydrophobe de manière à former une barrière à l'humidité, et la deuxième structure textile 21 étant néanmoins perméable à l'air et suffisamment respirante avec une résistance à la vapeur d'eau ($R_{et}$) en régime stationnaire inférieure à 6 m$^2$.Pa/W, afin de permettre une évacuation suffisamment efficace de la sueur à l'état de vapeur.

Plus particulièrement, la résistance à la vapeur d'eau ($R_{et}$) en régime stationnaire de la deuxième structure textile 21 est de préférence inférieure à 4,5 m$^2$.Pa/W, plus préférentiellement inférieure à 3,5 m$^2$.Pa/W, et encore plus préférentiellement inférieure à 2 m$^2$.Pa/W.

La mise en œuvre de cette deuxième structure textile 21 permet ainsi de limiter la formation d'auréoles dans l'article d'habillement 1, tout en permettant un séchage efficace de la première structure textile absorbante 20.

La mesure de la résistance à la vapeur d'eau ($R_{et}$) en régime stationnaire de la deuxième structure textile 21 est réalisée, de manière connue en soi, en mettant en œuvre la méthode de mesure de la norme NF EN ISO 11092 :2014 (« essai de la plaque chaude gardée transpirante »).

Dans le cadre de l'invention, ladite deuxième structure textile 21 peut comporter plusieurs couches textiles superposées, et notamment plusieurs tricots et/ou tissus superposés, dont au moins ledit deuxième tricot ou tissu 210.

Néanmoins, dans une variante préférée de réalisation, ladite deuxième structure textile 21 est constituée uniquement dudit deuxième tricot ou tissu barrière 210.

Le caractère hydrophobe du deuxième tricot ou tissu 210 est obtenu en utilisant au moins majoritairement des fils hydrophobes.

Dans ce cas, le deuxième tricot ou tissu 210 comporte de préférence au moins 50% en poids de fibres hydrophobes, et plus préférentiellement 100% de fibre hydrophobes.

Le deuxième tricot ou tissu 210 est par exemple constitué de fibres hydrophobes à base de polypropylène (de préférence au moins 50% en poids), et de préférence de fibres hydrophobes 100% polypropylène ou de fibres hydrophobes à base de polyester (de préférence au moins 50% en poids), et de préférence de fibres hydrophobes 100% polyester.

De préférence, les fils hydrophobes du deuxième tricot ou tissu 210 sont choisis de telle sorte que la deuxième structure textile 21 présente un taux de reprise mesuré inférieur à 3%.

Dans une variante préférée de réalisation, telle que celle illustrée par exemple sur la figure 2, la pièce protectrice 2 peut être constituée uniquement du premier tricot ou tissu 200 susvisé apte à absorber l'humidité et à réduire les odeurs de sueur et du deuxième tricot ou tissu 210 susvisé apte à former une barrière à l'humidité.

Dans une autre variante de réalisation, la pièce protectrice 2 peut comporter des couches textiles additionnelles superposées à partir du deuxième tricot 21, de manière à renforcer l'effet de barrière à l'humidité tout en maintenant pour la pièce protectrice 2, une perméabilité à l'air et une perméabilité suffisante à la vapeur d'eau. Dans ce cas la résistance à la vapeur d'eau ($R_{et}$) en régime stationnaire de chaque couche supplémentaire doit de préférence être inférieure 6 m$^2$.Pa/W.

De préférence, la pièce protectrice 2 est plus particulièrement dépourvue de couche supplémentaire (en plus des structures textiles susvisées 20 et 21) présentant une résistance à la vapeur d'eau ($R_{et}$) en régime stationnaire supérieure à 6 m$^2$.Pa/W. En particulier la pièce protectrice 2 est dépourvue de film ou membrane imper-respirant qui présente généralement une résistance à la vapeur d'eau ($R_{et}$) en régime stationnaire beaucoup trop importante, et typiquement nettement supérieure à 6 m$^2$.Pa/W selon la norme NF EN ISO 11092 :2014.

Les différentes couches textiles de la pièce protectrice 2 peuvent être préassemblées entre elles, avant montage ou positionnement de la pièce protectrice 2 dans l'article d'habillement 1, par exemple par une couture périphérique et/ou par un collage périphérique et/ou par des points de colle suffisamment petits et en faible nombre pour ne pas nuire à la perméabilité à l'air et au caractère respirant de la pièce protectrice 1.

L'assemblage des différentes couches textiles de la pièce protectrice 2 peut également être effectué lors du montage et de la fixation de la pièce protectrice 2 dans l'article d'habillement 1, la couture par exemple des différentes couches textiles avec l'article d'habillement 1 permettant également l'assemblage de ces couches textiles entre-elles.

<u>Exemples préférés de réalisation</u>

On a fabriqué des pièces protectrices 2 qui conformément à la variante préférée de réalisation de la figure 2 sont chacune constituées par :

- un premier tricot 200 (absorbant l'humidité et réduisant les odeurs) de type jersey vanisé ayant la structure de tricotage particulière de la figure 2,
  et

- un deuxième tricot 210 (barrière à l'humidité) de type jersey

Les deux tricots 200 et 210 sont liés ensemble par une couture périphérique.

Le fil hydrophile 2001 (figure 2) du premier tricot 200 est un fil 100% coton ayant numéro anglais (Ne) de 60 et un taux de reprise de l'ordre de 7% à 8% ; le fil hydrophobe 2002 (figure 2) du premier tricot 200 est un fil 100% polyester ayant un titrage de 55 dtex (50 deniers) et un taux de reprise inférieur à 2% ; le fil élastique 2003 (figure 2) est un fil d'élasthanne de titrage 22dtex (20 deniers). Le pourcentage en poids des différents fils du tricot 200 sont les suivants :

- fil 2001 (100% coton) : 62%
- fil 2002 (100 % polyester) : 33%
- fil 2003 (élasthanne) : 5%

Le deuxième tricot 210 est constitué d'un fil polypropylène hydrophobe, ayant titrage de 83 dtex (75 deniers) et ayant un taux de reprise mesuré inférieur à 3%.
Dans un premier échantillon A :

- le deuxième tricot 210 a une épaisseur de 0.4mm (mesurée sous une pression de 0,3KPa) et une masse de 99g/m$^2$;
- la pièce protectrice a une épaisseur de 0.71mm (mesurée sous une pression de 0,3KPa) et une masse de 163 g/m$^2$.

Dans un deuxième échantillon B :

- le deuxième tricot 210 a une épaisseur de 0.36mm (mesurée sous une pression de 0,3KPa) et une masse de 103g/m$^2$;
- la pièce protectrice a une épaisseur de 0.70mm (mesurée sous une pression de 0,3KPa) et une masse de 159 g/m$^2$.

Pour chaque échantillon A et B, le taux de réduction d'odeur de chaque premier tricot 200 est mesuré conformément à la méthode précédemment décrite (ISO 17299-3) de dosage de l'acide acétique par chromatographie en phase gazeuse.
Le taux de réduction d'odeur mesuré pour chaque premier tricot 200 de chaque échantillon A et B est de 79%.
Pour chaque échantillon A et B, la résistance à la vapeur d'eau ($R_{et}$) en régime stationnaire a été mesurée conformément à la méthode précédemment décrite dans la norme NF EN ISO 11092 :2014, séparément sur chaque tricot 200 et 210 avant assemblage ; les conditions climatiques pour la mesure de Ret étaient les suivantes : 35°C +/-5°C et 40%H.R +/-3%H.R.
La perméabilité à la vapeur d'eau a également été mesurée conformément à la norme NF EN ISO 11092 :2014 séparément sur chaque tricot 200 et 210 avant assemblage.
Les résultats de $R_{et}$ et de perméabilité à la vapeur d'eau sont synthétisés dans le tableau I suivant.

Tableau I : Ret et Perméabilité à la vapeur d'eau

| Échantillon | | $R_{et}$ (m$^2$.Pa/W) | Perméabilité à la vapeur d'eau (g/m$^2$.h.Pa) |
|---|---|---|---|
| A | 1$^{er}$ Tricot (200) | **2.69** | 0.553 |
| | 2$^{ème}$ Tricot (210) | **1.52** | 0.979 |
| B | 1$^{er}$ Tricot (200) | **2.62** | 0.568 |
| | 2$^{ème}$ Tricot (210) | **1.23** | 1.207 |

Pour chaque échantillon A et B, le taux de reprise de chaque tricot 200 et 210 a été mesuré conformément à la méthode ci-après.
Les échantillons sont conditionnés, à plat et dépliés, dans le laboratoire conditionné à 20°C et 65% HR pendant 24h. Après pesage (poids conditionné), ils sont mis dans une étuve à 105 °C pendant 16h minimum.
Ils sont ensuite placés rapidement dans des boîtes hermétiques ; ces dernières sont refroidies pendant 15 minutes dans un dessiccateur avec gel de silice, sous vide (trompe à eau).
Les boîtes froides sont pesées (boîte + échantillon), puis l'échantillon est retiré de la boîte et la boîte vide est pesée, de manière à obtenir le poids à sec de l'échantillon.
Les résultats de mesure du taux de reprise sont synthétisés dans le tableau II suivant.

Tableau II : Taux de reprise

| Échantillon | | Poids conditionnés (g) | Poids secs (g) | Perte de poids (9) | Perte de poids (%) | Taux de reprise |
|---|---|---|---|---|---|---|
| A | 1er Tricot (200) | 45,3470 | 42,3856 | 2,9614 | 6,53 | **6,99%** |
| | 2ème Tricot (210) | 17,9870 | 17,5069 | 0,4801 | 2,67 | **2,74%** |
| B | 1er Tricot (200) | 48,1850 | 45,3868 | 2,7982 | 5,81 | **6,17%** |
| | 2ème Tricot (210) | 31,7411 | 30,8637 | 0,8774 | 2,76 | **2,84%** |

Pour chaque échantillon A et B, la perméabilité à l'air de chaque tricot 200 et 210 avant assemblage et la perméabilité à l'air de la pièce protectrice 2 (tricot 200 et 210 cousus ensemble) ont été mesurées conformément à la norme NF EN ISO 9237 :1995 avec les conditions de mise en œuvre suivantes :

- Sens d'écoulement de l'air à travers l'étoffe : par aspiration
- Surface d'essai : 20 cm2
- Perte de charge : 100 Pa
- Nombre d'éprouvettes soumises à l'essai : 10
- Atmosphère de conditionnement : 20°C+/- 2°C et 65% +/-4% H.R
- Atmosphère de réalisation de la mesure: 20°C+/- 2°C et 65% +/-4% H.R Les résultats de mesure de la perméabilité à l'air sont synthétisés dans les tableaux III et IV suivants.

Tableau III : Échantillon A- Perméabilité à l'air

| Échantillon A | 1erTricot (200)/ Perméabilité à l'air (l/m$^2$/s) | 2èmeTricot (210) / Perméabilité à l'air (l/m$^2$/s) | Pièce protectrice (2)/ Perméabilité à l'air (l/m$^2$/s) |
|---|---|---|---|
| Éprouvette 1 | 386 | 1210 | 281 |
| Éprouvette 2 | 399 | 1250 | 273 |
| Éprouvette 3 | 358 | 1280 | 282 |
| Éprouvette 4 | 324 | 1240 | 276 |
| Éprouvette 5 | 326 | 1240 | 275 |
| Éprouvette 6 | 309 | 1220 | 286 |
| Éprouvette 7 | 314 | 1260 | 283 |
| Éprouvette 8 | 302 | 1240 | 280 |
| Éprouvette 9 | 308 | 1220 | 288 |
| Éprouvette 10 | 316 | 1240 | 279 |
| **Moyenne** | **334** | **1240** | **280** |

Tableau IV: Échantillon B - Perméabilité à l'air

| Échantillon B | 1erTricot (200)/ Perméabilité à l'air (l/m$^2$/s) | 2èmeTricot (210) / Perméabilité à l'air (l/m$^2$/s) | Pièce protectrice (2)/ Perméabilité à l'air (l/m$^2$/s) |
|---|---|---|---|
| Éprouvette 1 | 515 | 1270 | 395 |
| Éprouvette 2 | 517 | 1180 | 379 |
| Éprouvette 3 | 513 | 1220 | 385 |

(suite)

| Échantillon B | 1$^{er}$Tricot (200)/ Perméabilité à l'air (l/m$^2$/s) | 2$^{ème}$Tricot (210) / Perméabilité à l'air (l/m$^2$/s) | Pièce protectrice (2)/ Perméabilité à l'air (l/m$^2$/s) |
|---|---|---|---|
| Éprouvette 4 | 518 | 1170 | 386 |
| Éprouvette 5 | 493 | 1230 | 389 |
| Éprouvette 6 | 504 | 1240 | 381 |
| Éprouvette 7 | 552 | 1270 | 361 |
| Éprouvette 8 | 508 | 1210 | 381 |
| Éprouvette 9 | 463 | 1170 | / |
| Éprouvette 10 | 481 | 1160 | / |
| **Moyenne** | **506** | **1212** | **382** |

## Revendications

1. Pièce protectrice (2) destinée à être intégrée à un article d'habillement (1), et notamment à un sous-vêtement, ladite pièce protectrice (2) étant apte à absorber la transpiration, à réduire les odeurs de sueur, et à limiter l'apparition d'auréoles, ladite pièce protectrice (2) étant perméable à l'air et au moins à la vapeur d'eau et comportant deux structures textiles (20 , 21) superposées :

   - une première structure textile (20) absorbante comportant au moins un premier tricot ou tissu (200) absorbant, dont la face interne (200a) constitue la face interne (2a) de la pièce protectrice destinée à être en contact avec la peau, et qui comporte sur sa face interne (200a) majoritairement des fils hydrophiles (2001) aptes à absorber l'humidité et à réduire les odeurs de sueur, ladite première structure textile absorbante présentant un taux de réduction d'odeur, mesuré par la méthode ISO 17299-3 de dosage de l'acide acétique par chromatographie en phase gazeuse, qui est supérieur à 50%,
   et
   - une deuxième structure textile (21) formant une barrière à l'humidité et comportant au moins un deuxième tricot ou tissu (210) hydrophobe en contact avec ladite première structure textile absorbante (20), ladite deuxième structure textile (21) étant perméable à l'air et respirante avec une résistance à la vapeur d'eau (Ret) en régime stationnaire, mesurée selon la norme NF EN ISO 11092:2014, inférieure à 6m$^2$.Pa/W,

   ladite pièce protectrice étant dépourvue de couche supplémentaire présentant une résistance à la vapeur d'eau (R$_{et}$) en régime stationnaire, mesurée selon la norme NF EN ISO 11092 :2014, supérieure à 6m$^2$.Pa/W.

2. Pièce protectrice selon la revendication 1, dans laquelle lesdits fils hydrophiles (2001) aptes à absorber l'humidité et à réduire les odeurs de sueur comportent des fils comportant une ou plusieurs matières naturelles hydrophiles, et de préférence au moins 50% en poids de une ou plusieurs matières naturelles hydrophiles, et plus préférentiellement encore 100% en poids de matières naturelles hydrophiles.

3. Pièce protectrice selon la revendication 2, dans laquelle la ou les matières naturelles hydrophiles sont choisies parmi les matières de la liste suivante : coton, lin, soie, laine, ou un mélange d'au moins deux de ces matières.

4. Pièce protectrice selon l'une quelconque des revendications précédentes, dans laquelle lesdits fils hydrophiles (2001) aptes à absorber l'humidité et à réduire les odeurs de sueur comportent des fils synthétiques traités hydrophiles, et de préférence dans laquelle lesdits fils hydrophiles (2001) ou le premier tricot ou tissu (200) ont été traités avec au moins un agent bactériostatique.

5. Pièce protectrice selon l'une quelconque des revendications 3 ou 4, dans laquelle lesdits fils hydrophiles (2001) aptes à absorber l'humidité et à réduire les odeurs de sueur comportent des fils comportant du polyester chargé en carbone, et de préférence chargé en charbon de bambou, de préférence des fils comportant au moins 50% en poids de polyester chargé en carbone, et plus préférentiellement des fils comportant 100% en poids de polyester chargé en carbone.

**6.** Pièce protectrice selon l'une quelconque des revendications précédentes, dans laquelle ledit premier tricot ou tissu (200) absorbant comporte majoritairement sur sa face interne (200a) lesdits fils hydrophiles (2001) aptes à absorber l'humidité et à réduire les odeurs de sueur, et comporte majoritairement sur son autre face externe (200b) des fils hydrophobes (2002), de manière à former une première barrière à l'humidité en complément de la deuxième structure textile (21), et de préférence dans laquelle lesdits fils hydrophobes présents majoritairement sur la face externe (200b) du premier tricot ou tissu (200) absorbant comportent des fils hydrophobes à base de polyester, et plus préférentiellement encore des fils 100% polyester.

**7.** Pièce protectrice selon l'une quelconque des revendications précédentes, dans laquelle ladite première structure textile (20) absorbante présente un taux de réduction d'odeur supérieur à 60%, de préférence supérieur à 70%.

**8.** Pièce protectrice selon l'une quelconque des revendications précédentes, dans laquelle ladite première structure textile (20) absorbante est constituée uniquement dudit premier tricot ou tissu (200) absorbant, et/ou dans laquelle ladite deuxième structure textile (21) formant une barrière à l'humidité est constituée uniquement dudit deuxième tricot ou tissu (210) hydrophobe.

**9.** Pièce protectrice selon l'une quelconque des revendications précédentes, constituée uniquement de ladite première structure textile (20) absorbante et de ladite deuxième structure textile (21) formant une barrière à l'humidité, et de préférence constituée uniquement dudit premier tricot ou tissu (200) absorbant et dudit deuxième tricot ou tissu (210) hydrophobe.

**10.** Pièce protectrice selon l'une quelconque des revendications précédentes, dans laquelle le premier tricot ou tissu (200) absorbant comporte au moins un fil élastique (2003), et plus particulièrement un fil en élasthanne.

**11.** Pièce protectrice selon l'une quelconque des revendications précédentes, dans laquelle la résistance à la vapeur d'eau (Ret) en régime stationnaire, mesurée selon la norme NF EN ISO 11092:2014, de ladite deuxième structure textile (21) est inférieure à 4,5 $m^2$.Pa/W, de préférence inférieure à 3,5 $m^2$.Pa/W, et plus préférentiellement inférieure à 2 $m^2$.Pa/W et/ou dans laquelle la première structure textile (20) absorbante présente une résistance à la vapeur d'eau ($R_{et}$) en régime stationnaire, mesurée selon la norme NF EN ISO 11092:2014, inférieure à 6$m^2$.Pa/W, de préférence inférieure à 4,5 $m^2$.Pa/W, et plus préférentiellement inférieure à 3 $m^2$.Pa/W.

**12.** Pièce protectrice selon l'une quelconque des revendications précédentes, dans laquelle la première structure textile (20) absorbante présente un taux de reprise supérieur à 4%, de préférence supérieur à 5%, et plus préférentiellement encore supérieur à 6% et/ou dans laquelle la deuxième structure textile (21) présente un taux de reprise mesuré inférieur à 3%.

**13.** Pièce protectrice selon l'une quelconque des revendications précédentes, dans laquelle la première structure textile (20) absorbante a une perméabilité à l'air supérieure à 100 l/$m^2$/s, et de préférence supérieure à 200 l/$m^2$/s et/ou dans laquelle la deuxième structure textile (21) a une perméabilité à l'air supérieure à 300 l/$m^2$/s, de préférence supérieure à 600 l/$m^2$/s, et plus préférentiellement encore supérieure à 800 l/$m^2$/s et/ou ladite pièce protectrice ayant une perméabilité à l'air supérieure à 100 l/$m^2$/s, et de préférence supérieure à 200 l/$m^2$/s.

**14.** Pièce protectrice selon l'une quelconque des revendications précédentes, dans laquelle le deuxième tricot ou tissu (210) hydrophobe comporte des fils hydrophobes synthétiques, notamment des fils à base polypropylène, de préférence des fils 100% polypropylène et/ou des fils à base de polyester, de préférence des fils 100% polyester.

**15.** Article d'habillement (1), et notamment sous-vêtement, dans lequel est intégrée, de manière amovible ou non amovible, au moins une pièce protectrice (2) de l'une quelconque des revendications précédentes, de telle sorte que la face interne (200a) du premier tricot ou tissu (200) absorbant de la pièce protectrice (2) peut être en contact avec la peau.

**16.** Article d'habillement, pour partie haute du corps, dans lequel sont intégrées, de manière amovible ou non amovible, au niveau respectivement des deux aisselles, deux pièces protectrices axillaires (2) de l'une quelconque des revendications 1 à 14, formant des pièces protectrices axillaires de telle sorte que la face interne (200a) du premier tricot ou tissu (200) absorbant de chaque pièce protectrice axillaire (2) peut être en contact avec la peau.

**Patentansprüche**

1. Schutzteil (2) zur Integration in ein Kleidungsstück (1) und insbesondere in Unterwäsche, wobei das Schutzteil (2) in der Lage ist, Schweiß zu absorbieren, Schweißgerüche zu verringern und das Auftreten von Schweißrändern einzuschränken, wobei das Schutzteil (2) durchlässig für Luft und zumindest Wasser ist und zwei übereinanderliegende Textilstrukturen (20, 21) aufweist:

   - eine erste absorbierende Textilstruktur (20), welche mindestens ein erstes, absorbierendes Gewirk/Gestrick oder Gewebe (200) umfasst, dessen Innenseite (200a) die Innenseite (2a) des Schutzteils, die mit der Haut in Kontakt kommt, bildet, und welche auf deren Innenseite (200a) mehrheitlich hydrophile Fäden (2001) aufweist, die in der Lage sind, Feuchtigkeit zu absorbieren und Schweißgerüche zu verringern, wobei die erste absorbierende Textilstruktur eine Geruchsreduzierungsrate, gemessen durch das Verfahren ISO 17299-3 unter Zudosierung von Essigsäure durch Gaschromatographie, aufweist, die größer als 50 % ist, und
   - eine zweite Textilstruktur (21), welche eine Feuchtigkeitsbarriere bildet und mindestens ein zweites, hydrophobes Gewirk/Gestrick oder Gewebe (210) umfasst, das mit der ersten absorbierenden Textilstruktur (20) in Kontakt ist, wobei die zweite Textilstruktur (21) luftdurchlässig und atmungsaktiv ist mit einem Wasserdampfwiderstand (Ret) im stationären Zustand, gemessen gemäß der Norm NF EN ISO 11092:2014, von weniger als 6 m$^2$.Pa/W,

   wobei das Schutzteil keine zusätzliche Schicht mit einem Wasserdampfwiderstand ($R_{et}$) im stationären Zustand, gemessen nach der Norm NF EN ISO 11092:2014, von mehr als 6 m$^2$.Pa/W aufweist.

2. Schutzteil nach Anspruch 1, wobei die hydrophilen Fäden (2001), die in der Lage sind, Feuchtigkeit zu absorbieren und Schweißgerüche zu verringern, Fäden aufweisen, die ein oder mehrere natürliche hydrophile Materialien umfassen, bevorzugt mindestens 50 Gew.-% eines oder mehrerer natürlicher hydrophiler Materialien, und weiter bevorzugt 100 Gew.-% natürlicher hydrophiler Materialien.

3. Schutzteil nach Anspruch 2, wobei das natürliche hydrophile Material oder die hydrophilen natürlichen Materialien aus der folgenden Liste ausgewählt ist/sind: Baumwolle, Leinen, Seide, Wolle oder einer Mischung aus mindestens zwei dieser Materialien.

4. Schutzteil nach einem der vorhergehenden Ansprüche, wobei die hydrophilen Fäden (2001), die in der Lage sind, Feuchtigkeit zu absorbieren und Schweißgerüche zu verringern, hydrophil behandelte synthetische Fäden umfassen, und wobei bevorzugt die hydrophilen Fäden (2001) oder das erste Gewirk/Gestrick oder Gewebe (200) mit mindestens einem bakteriostatischen Mittel behandelt worden sind.

5. Schutzteil nach einem der Ansprüche 3 oder 4, wobei die hydrophilen Fäden (2001), die in der Lage sind, Feuchtigkeit zu absorbieren und Schweißgerüche zu verringern, Fäden umfassen, die kohlenstoffbeladenen Polyester aufweisen, bevorzugt mit Bambuskohle beladenen Polyester, bevorzugt Fäden, die mindestens 50 Gew.-% kohlenstoffbeladenen Polyester aufweisen, und noch bevorzugter Fäden, die 100 Gew.-% kohlenstoffbeladenen Polyester aufweisen.

6. Schutzteil nach einem der vorhergehenden Ansprüche, wobei das erste, absorbierendes Gewirk/Gestrick oder Gewebe (200) mehrheitlich auf dessen Innenseite (200a) die genannten hydrophilen Fäden (2001) aufweist, die in der Lage sind, Feuchtigkeit zu absorbieren und Schweißgerüche zu verringern, und mehrheitlich auf seiner anderen, äußeren Seite (200b) hydrophobe Fäden (2002) aufweist, so dass eine erste Feuchtigkeitsbarriere zusätzlich zu der zweiten Textilstruktur (21) gebildet wird, und wobei bevorzugt die hydrophoben Fäden, die mehrheitlich auf der Außenseite (200b) des ersten, absorbierenden Gewirks/Gestricks oder Gewebes (200) vorhanden sind, hydrophobe Fäden auf Polyesterbasis umfassen und noch bevorzugter 100% Polyesterfäden umfassen.

7. Schutzteil nach einem der vorhergehenden Ansprüche, wobei die erste absorbierende Textilstruktur (20) eine Geruchsreduktionsrate von mehr als 60%, vorzugsweise mehr als 70%, aufweist.

8. Schutzteil nach einem der vorhergehenden Ansprüche, wobei die erste absorbierende Textilstruktur (20) ausschließlich aus dem ersten, absorbierenden Gewirk/Gestrick oder Gewebe (200) gebildet ist, und/oder wobei die zweite Textilstruktur (21), die eine Feuchtigkeitsbarriere bildet, ausschließlich aus dem zweiten, hydrophoben Gewirk/Gestrick oder Gewebe (210) gebildet ist.

9. Schutzteil nach einem der vorhergehenden Ansprüche, das ausschließlich aus der ersten absorbierenden Textilstruktur (20) und der eine Feuchtigkeitsbarriere bildenden zweiten Textilstruktur (21) gebildet ist, und bevorzugt ausschließlich aus dem ersten, absorbierenden Gewirk/Gestrick oder Gewebe (200) und dem zweiten, hydrophoben Gewirk/Gestrick oder Gewebe (210) gebildet ist.

10. Schutzteil nach einem der vorhergehenden Ansprüche, wobei das erste, absorbierende Gewirk/Gestrick oder Gewebe (200) mindestens ein elastisches Garn (2003), insbesondere ein Elastan-Garn, aufweist.

11. Schutzteil nach einem der vorhergehenden Ansprüche, bei dem der Wasserdampfwiderstand ($R_{et}$) im stationären Zustand, gemessen nach der Norm NF EN ISO 11092:2014, der zweiten Textilstruktur (21) geringer als 4,5 m$^2$.Pa/W, bevorzugt geringer als 3,5 m$^2$.Pa/W und weiter bevorzugt geringer als 2.m$^2$Pa/W ist, und/oder bei dem die erste absorbierende Textilstruktur (20) einen Wasserdampfwiderstand (Ret) im stationären Zustand, gemessen nach der Norm NF EN ISO 11092:2014, von weniger als 6 m$^2$.Pa/W, bevorzugt von weniger als 4,5 m$^2$.Pa/W und weiter bevorzugt von weniger als 3 m$^2$.Pa/W aufweist.

12. Schutzteil nach einem der vorhergehenden Ansprüche, wobei die erste absorbierende Textilstruktur (20) ein Rückstellungsverhältnis von mehr als 4%, bevorzugt mehr als 5% und noch weiter bevorzugt mehr als 6% aufweist, und/oder wobei die zweite Textilstruktur (21) ein gemessenes Rückstellungsverhältnis von weniger als 3% aufweist.

13. Schutzteil nach einem der vorhergehenden Ansprüche, wobei die erste absorbierende Textilstruktur (20) eine Luftdurchlässigkeit von mehr als 100 l/m$^2$/s und bevorzugt mehr als 200 l/m$^2$/s aufweist, und/oder wobei die zweite Textilstruktur (21) eine Luftdurchlässigkeit von mehr als 300l/m$^2$/s, bevorzugt mehr als 600 l/m$^2$/s, und noch weiter bevorzugt mehr als 800 l/m$^2$/s aufweist, und/oder wobei das Schutzteil eine Luftdurchlässigkeit von mehr als 100 l/m$^2$/s und bevorzugt mehr als 200 l/m$^2$/s aufweist.

14. Schutzteil nach einem der vorhergehenden Ansprüche, wobei das zweite, hydrophobe Gewirk/Gestrick oder Gewebe (210) synthetische hydrophobe Fäden, insbesondere Fäden auf Polypropylenbasis, bevorzugt 100% Polypropylenfäden, und/oder Fäden auf Polyesterbasis, bevorzugt 100 % Polyesterfäden, umfasst.

15. Kleidungsstück (1), insbesondere Unterwäsche, bei dem mindestens ein Schutzteil (2) nach einem der vorhergehenden Ansprüche entfernbar oder nicht entfernbar derart integriert ist, dass die Innenseite (200a) des ersten, absorbierenden Gewirks/Gestricks oder Gewebes (200) des Schutzteils (2) mit der Haut in Kontakt sein kann.

16. Kleidungsstück für den Oberkörper, bei dem zwei Achsel-Schutzteile (2) nach einem der Ansprüche 1 bis 14 jeweils auf Höhe der beiden Achselhöhlen entfernbar oder nicht entfernbar integriert sind, wobei die Achsel-Schutzteile derart gebildet sind, dass die Innenfläche (200a) des ersten, absorbierenden Gewirks/Gestricks oder Gewebes (200) jedes Achsel-Schutzteils (2) mit der Haut in Kontakt sein kann.

**Claims**

1. Protective piece (2) intended for being integrated in an article of clothing (1), in particular underwear, said protective piece (2) being capable of absorbing perspiration, reducing sweat odors, and limiting the appearance of sweat marks, said protective piece (2) being permeable to air and at least to water vapor and comprising two textile structures (20, 21) one on top of the other:

   - a first absorbent textile structure (20) comprising at least a first absorbent knit or fabric (200), the internal face (200a) of which constitutes the internal face (2a) of the protective piece intended for being in contact with the skin, and which comprises, on its internal face (200a), mainly hydrophilic yarns (2001) capable of absorbing moisture and reducing sweat odors, said first absorbent textile structure having an odor reduction rate, measured by the ISO 17299-3 method for determining acetic acid by gas chromatography, of greater than 50%, and
   - a second textile structure (21) forming a moisture barrier and comprising at least a second hydrophobic knit or fabric (210) in contact with said first absorbent textile structure (20), said second textile structure (21) being permeable to air and breathable with a water-vapor resistance ($R_{et}$) under steady state conditions, measured according to standard NF EN ISO 11092:2014, of less than 6 m$^2$.Pa/W,

said protective piece being free of an additional layer having a water-vapor resistance ($R_{et}$) under steady state

conditions, measured according to standard NF EN ISO 11092:2014, of greater than 6 $m^2$.Pa/W.

2. Protective piece according to claim 1, wherein said hydrophilic yarns (2001) capable of absorbing moisture and reducing sweat odors comprise yarns comprising one or more natural hydrophilic materials, preferably at least 50 wt.% of one or more natural hydrophilic materials, more preferably even 100 wt.% of natural hydrophilic materials.

3. Protective piece according to claim 2, wherein the natural hydrophilic material(s) are selected from among the materials in the following list: cotton, linen, silk, wool, or a mixture of at least two of these materials.

4. Protective piece according to any of the preceding claims, wherein said hydrophilic yarns (2001) capable of absorbing moisture and reducing sweat odors comprise treated synthetic hydrophilic yarns, and preferably wherein said hydrophilic yarns (2001) or the first knit or fabric (200) have been treated with at least one bacteriostatic agent.

5. Protective piece according to either claim 3 or claim 4, wherein said hydrophilic yarns (2001) capable of absorbing moisture and reducing sweat odors comprise yarns comprising polyester loaded with carbon, preferably loaded with bamboo charcoal, preferably yarns comprising at least 50 wt.% of polyester loaded with carbon, more preferably yarns comprising 100 wt.% of polyester loaded with carbon.

6. Protective piece according to any of the preceding claims, wherein said first absorbent knit or fabric (200) comprises mainly said hydrophilic yarns (2001) capable of absorbing moisture and reducing sweat odors on its internal face (200a), and comprises mainly hydrophobic yarns (2002) on its other external face (200b), so as to form a first moisture barrier in addition to the second textile structure (21), and preferably wherein said hydrophobic yarns present mainly on the external face (200b) of the first absorbent knit or fabric (200) comprise hydrophobic polyester-based yarns, more preferably even 100% polyester yarns.

7. Protective piece according to any of the preceding claims, wherein said first absorbent textile structure (20) has an odor reduction rate of greater than 60%, preferably greater than 70%.

8. Protective piece according to any of the preceding claims, wherein said first absorbent textile structure (20) consists only of said first absorbent knit or fabric (200), and/or wherein said second textile structure (21) forming a moisture barrier consists only of said second hydrophobic knit or fabric (210).

9. Protective piece according to any of the preceding claims, consisting only of said first absorbent textile structure (20) and of said second textile structure (21) forming a moisture barrier, preferably consisting only of said first absorbent knit or fabric (200) and said second hydrophobic knit or fabric (210).

10. Protective piece according to any of the preceding claims, wherein the first absorbent knit or fabric (200) comprises at least one elastic yarn (2003), more particularly an elastane yarn.

11. Protective piece according to any of the preceding claims, wherein the water-vapor resistance ($R_{et}$) under steady state conditions, measured according to standard NF EN ISO 11092:2014, of said second textile structure (21) is less than 4.5 $m^2$.Pa/W, preferably less than 3.5 $m^2$.Pa/W, more preferably less than 2 $m^2$.Pa/W, and/or wherein the first absorbent textile structure (20) has a water-vapor resistance ($R_{et}$) under steady state conditions, measured according to standard NF EN ISO 11092:2014, of less than 6 $m^2$.Pa/W, preferably less than 4.5 $m^2$.Pa/W, more preferably less than 3 $m^2$.Pa/W.

12. Protective piece according to any of the preceding claims, wherein the first absorbent textile structure (20) has a regain rate of greater than 4%, preferably greater than 5%, more preferably even greater than 6%, and/or wherein the second textile structure (21) has a measured regain rate of less than 3%.

13. Protective piece according to any of the preceding claims, wherein the first absorbent textile structure (20) has an air permeability of greater than 100 l/$m^2$/s, preferably greater than 200 l/$m^2$/s, and/or wherein the second textile structure (21) has an air permeability of greater than 300 l/$m^2$/s, preferably greater than 600 l/$m^2$/s, and more preferably even greater than 800 l/$m^2$/s, and/or said protective piece having an air permeability of greater than 100 l/$m^2$/s, preferably greater than 200 l/$m^2$/s.

14. Protective piece according to any of the preceding claims, wherein the second hydrophobic knit or fabric (210) comprises synthetic hydrophobic yarns, in particular yarns based on polypropylene, preferably 100% polypropylene yarns, and/or yarns based on polyester, preferably 100% polyester yarns.

15. Article of clothing (1), in particular underwear, in which at least one protective piece (2) according to any of the preceding claims is integrated in a removable or non-removable manner, such that the internal face (200a) of the first absorbent knit or fabric (200) of the protective piece (2) can be in contact with the skin.

16. Article of clothing for the upper part of the body in which two axillary protective pieces (2) according to any of claims 1 to 14 are integrated in a removable or non-removable manner, at the two armpits, respectively, which pieces forming axillary protective pieces such that the internal face (200a) of the first absorbent knit or fabric (200) of each axillary protective piece (2) can be in contact with the skin.

20(200)

21(210)

10

2

2a(200a)

200b

1

2

2

10

2

## FIG.1

FIG.2

## RÉFÉRENCES CITÉES DANS LA DESCRIPTION

**Documents brevets cités dans la description**

- WO 2015148758 A
- WO 2010004175 A
- FR 3026923